(19) Europäisches Patentamt
European Patent Office
Office européen des brevets

(11) Numéro de publication: **0 298 796**
**A1**

(12) # DEMANDE DE BREVET EUROPEEN

(21) Numéro de dépôt: **88401460.6**

(22) Date de dépôt: **14.06.88**

(51) Int. Cl.$^4$: **C 12 P 13/02**
C 12 P 17/00, C 12 P 17/10,
C 12 P 17/14

(30) Priorité: **17.06.87 FR 8708460**

(43) Date de publication de la demande:
**11.01.89 Bulletin 89/02**

(84) Etats contractants désignés: **DE GB IT NL**

(71) Demandeur: **SOCIETE D'EXPLOITATION DE PRODUITS POUR LES INDUSTRIES CHIMIQUES (S.E.P.P.I.C.)**
**70, Champs-Elysées**
**F-75008 Paris (FR)**

(72) Inventeur: **Graille, Jean**
**1373 Avenue Père Soulas**
**F-3400 Montpellier (FR)**

**Montet, Didier**
**Résidence des Cyclamens A-25 11 Rue des Bleuts**
**F-34070 Montpellier (FR)**

**Servat, Françoise**
**35 Avenue de la Colline**
**F-34070 Montpellier (FR)**

**Grimaud, Jean**
**134 Rue du Curat**
**F-34100 Montpellier (FR)**

**Renard, Gilbert**
**11 Les Claperèdes**
**F-34270 Saint Mathieu de Treviers (FR)**

**Galzy, Pierre**
**1 Rue des Mélèzes**
**F-34100 Montpellier (FR)**

**Arnaud, Alain**
**Cours de la Chicane**
**F-34800 Clermont L'Herault (FR)**

**Marcou, Lucien**
**11 Avenue Marinville**
**F-94100 Saint Maur (FR)**

(74) Mandataire: **Combe, André et al**
**CABINET BEAU DE LOMENIE 55, rue d'Amsterdam**
**F-75008 Paris (FR)**

(54) **Synthèse d'amides gras N-substitués par catalyse enzymatique.**

(57) La présente invention concerne un procédé pour la synthèse d'amides gras N-substitués par réaction d'un composé organique présentant une fonction acide terminale et un composé organique présentant une fonction amine terminale caractérisé en ce que ladite réaction est effectuée en présence d'une enzyme de la classe des acyltransférases.

EP 0 298 796 A1

**Description**

**Synthèse d'amides gras N-substitués par catalyse enzymatique**

L'invention concerne un procédé de synthèse d'amides N-substitués consistant à faire réagir au moins un acide organique dont la condensation en carbone s'étale de $C_2$ à $C_{40}$ avec un synthon possédant au moins une fonction amine et éventuellement une ou plusieurs autres fonctions (alcool, thiol, fonction phosphorée amide, ester, acide, etc.) ; il est à noter que l'acide organique peut également posséder une ou plusieurs de ces fonctions.

Dans certains solvants polaires protiques, tels les alcools tertiaires, les synthons aminés difonctionnels à deux ou trois carbones comme l'éthanolamine, l'éthylènediamine, l'éthanethiolamine, l'amino-1-propanol-3, l'amino éthyl éthanolamine, la propyldiamine-1,3 et l'amino-1-propanethiol-3, on obtient des hétérocycles à cinq ou six atomes dont les hétéroatomes sont en position 1-3, les cycles étant substitués en 2. On obtient ainsi respectivement des Δ2-oxazolines, des Δ2-imidazolines, des Δ2-thiazolines-1-3 des Δ2-morpholines-1-3 des Δ2-pyrimidines-1-3 et des Δ2-thiazines-1-3 ; tous ces composés substitués en position 2 présentent un intérêt particulier en terme d'amphiphilie lorsque R est une longue chaîne aliphatique suivant les deux schémas ci-après.

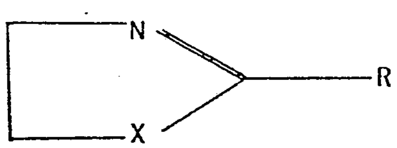

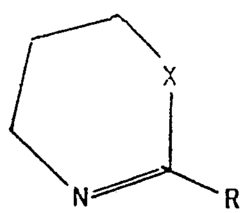

X = O, S ou N R compte un atome de carbone en moins par rapport à l'acide mis en jeu. La formation de ces cycles est due essentiellement à l'effet du solvant et à la configuration des synthons aminés faborable à la cyclisation.

Ces exemples ne sont pas restrictifs.

Etat de la technique :

L'utilisation d'enzymes pour la synthèse organique est devenue aujourd'hui un champ pluridisciplinaire très vaste rassemblant de nombreux chercheurs et suscitant un intérêt grandissant au fil des années de la part du monde industriel.

Il est à remarquer que,dans la plupart des réactions décrites dans la littérature, les enzymes sont mises en oeuvre pour les réactions très voisines de celles pour lesquelles la nature les a destinées in vivo.

Les progrès enregistrés aujourd'hui résultent d'une observation qui consiste en ce que les catalyseurs biologiques sont aptes à catalyser des réactions in vitro qui n'ont plus rien à voir avec les réactions pour lesquelles ils étaient programmés in vivo; de même on doit ajouter que les conditions de mise en oeuvre s'écartent très souvent radicalement de celles in vivo : les enzymes sont utilisées en fait comme de simples catalyseurs de la chimie organique, à la différence fondamentale près que les catalyseurs biologiques sont très souvent spécifiques, régiosélectifs ou énantiosélectifs pour des réactions pour lesquelles ils n'étaient pas programmés in vivo.

La réaction d'un acide avec une amine catalysée par une enzyme peut être schématisée de la manière suivante :

$$R\ COOH + R'NH \overset{E}{\rightleftharpoons} R - CONH - R' + H_2O$$

E = enzyme

La réaction est déplacée quantitativement vers la synthèse en piégeant en continu l'eau formée.

Les enzymes mises en oeuvre sont des acyltransférases :

- Les lipases
- Les acylases
- Les peptidases
- Les protéinases
- Les amidases

Ces enzymes sont utilisées industriellement quasiment exclusivement dans le sens de l'hydrolyse.

Citons par exemple l'emploi de la papaïne, enzyme protéolytique, dans l'industrie de la bière (clarification), dans l'industrie des cuirs (assouplissement et nettoyage), en boucherie industrielle (hydrolyse de l'élastine

pour attendrir les viandes), en diététique hospitalière pour la préparation d'aliments protéiques prédigérés.

Citons dans l'industrie pharmaceutique l'utilisation de la pénicilline V acylase qui catalyse l'hydrolyse complète de la fonction amide de la phénoxypénicilline (penicilline V) en acide amino-6-pénicillanique (6 APA) aet en acide phénoxyacétique.

Citons la production d'acides gras de haute qualité par hydrolyse de corps gras à l'aide d'une lipase fixée.

Citons enfin l'hydrolyse de l'acrylonitrile en acrylamide ou en acide acrylique.

Les réactions d'hydrolyse sont les plus répandues car ce sont les plus faciles à mettre en oeuvre aussi bien sur le plan technique qu'économique ; en effet ces enzymes ne nécessitent pas de co-facteurs, biomolécules très coûteuses et difficiles à recycler quantitativement.

Les réactions inverses de synthèse sont également promises a un avenir brillant car un bon nombre de molécules sont inaccessibles ou d'une accession trop coûteuse par les voies classiques de la chimie organique.

La synthèse d'amdes N-substitués revêt un intérêt tout particulièr pour l'industrie des amphiphiles dans des domaines aussi divers que les produits tensioactifs : les émulsifiants, les agents mouillants, anticorrosion, adoucissants, etc., ces molécules pouvant trouver des applications aussi bien industrielles, ménagères, cosmétiques que pharmaceutiques.

Ce type de molécules peut en fait couvrir une grande plage HLB. A notre connaissance aucun brevet n'a décrit la synthèse enzymatique de tels composés : seules trois publications connues de nous font allusion à des transferts d'acyles sur l'atome d'azote.

1) "Ester synthesis catalysed by polyethylene glycol-modified lipase in benzene".

Y. Inada, H. Nishimura, K.Takahasshi, T. Yoshimoto, A. Ranjan Saha and Y. Saito
Biochemical and Biophysical Research Communications, (1984), 122, 845-850

Dans ce document les auteurs font réagir la laurylamine sur le laurylstéarate pour obtenir le N-laurylstéaramide et l'alcool laurique ; c'est une réaction d'aminolyse d'ester.

2) "Synthesis of esters by lipases "

G. Lazar, A. Weiss and R.D. Schmid.
World conference on emerging technologies in the Fats and Oils Industry. AOCS Congress Cannes 3-8/11/1985, 346-354

Dans ce document les auteurs décrivent l'acylation de composés soufrés ou azotés en milieu aqueux. Les résultats sont consignés dans le tableau suivant :
Estérification enzymatique de l'acide oléique avec des composés contenant des atomes d'azote ou de soufre.

| Réactif | Lipase(s)<br>Mucor spec LK 13 | Pseudomonas spec LK 15 | Rhizopus delemar LK 31 |
|---|---|---|---|
| | Rendement de la synthèse (%) | | |
| éthanolamine | 0 | 0 | 0 |
| triéthanolamine | 0 | 0 | 0 |
| 2-mercaptoéthanol | 20 | 30 | 30 |
| éthanethiol | 10 | 10 | 5 |
| 2-mercaptoéthylamine HCL | 0 | 0 | 0 |
| L-cystéine | 0 | 0 | 0 |
| L-cystéine HCL | 0 | 0 | 0 |

On constate que ces auteurs n'ont pas réussi à acyler les fonctions amines de leur substrats.

3) "Hydrolyse et synthèse de liaison ester par la lipase d'un mycélium dévitalisé de Rhizopus arrhizus en milieu non aqueux"

C. GANCET et C. GRIGNARD

Revue Française des Corps Gras, 1986, 33, 423-430

Dans les conclusions de cet article les auteurs écrivent :

"Il faut également considérer que ce type de catalyse peut s'appliquer à d'autres réactions, non seulement à la synthèse d'esters comme cela vient d'être décrit, mais également à la création de liaison amide, thio-ester et thioamide".

Ces auteurs n'ont à ce jour publié aucun article ni brevet sur ces types de réactions.

Objet de l'invention

Les acides organiques utilisés et les acides gras, à la différence de ce qui est suggéré ou obtenu dans la littérature, réagissent avec des rendements importants sur les fonctions amines ; suivant les conditions opératoires, lorsque la fonction amine est en compétition avec d'autres fonctions, les acides réagissent sur la fonction amine avec une grande sélectivité.

En particulier et à titre d'exemple, lorsque l'on fait réagir les acides sur des aminopropanols, on obtient exclusivement les N-acyl-aminopropanols mais, dans certaines, conditions on obtient des composés cycliques.

Description générale de l'invention

Selon l'invention le procédé est caractérisé en ce qu'il consiste à mettre en contact sous agitation l'acide et l'amine suivant les cas :

- En phase homogène dans un solvant organique, l'enzyme étant dispersée dans le milieu soit en l'état, soit fixée sur un support de synthèse ou biologique (cellules du micro-organisme synthétisant l'enzyme).
- En phase hétérogène comprenant une phase organique et une phase aqueuse limitée ou importante.
- En milieu homogène fondu c'est-à-dire sans solvant organique.
- En milieu aqueux hétérogène sans solvant organique.

Suivant le cas l'enzyme libre est en solution dans la phase aqueuse qui peut être tamponnée. Les réactions peuvent être effectuées en batch ou sur colonne à lit catalytique fixe suivant le cas.

Dans son aspect le plus général la réaction peut être appliquée à de nombreuses molécules d'amines ou d'acides.

On peut citer parmi ces molécules, bien entendu à titre d'exemple non restrictif, les amines linéaires de formule $C_nH_{2n+1}\,NH_2$ où n est compris entre 2 et 30, les amines ramifiées, les amines insaturées; les diamines comme l'éthylène diamine, la diéthylène diamine et les hydrazines ; les amino alcools comme les amino propanols et les hydroxylamines ($NH_2\,OH$ et les dérivés N ou O substitués) ; citons également les acides aminés naturels ou de synthèse, les amines aromatiques et toute amine possédant sur sa molécule une ou plusieurs fonctions organiques comme cité précédemment. Mais l'invention n'est pas limitée à l'utilisation d'amines dans lesquelles la fonction amine est en bout de chaîne. Des résultats positifs (formation d'amide) ont également été obtenus avec des produits tels que

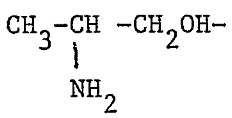

Bien entendu on peut également utiliser sans aucune restriction les mélanges de deux ou de plusieurs de ces amines.

Parmi les acides organiques, on peut citer les acides linéaires ou ramifiés, saturés ou insaturés, et par exemple les acides gras saturés ou insaturés.

L'acide peut présenter dans sa molécule d'autres fonctions organiques comme indiqué précédemment. Citons à titre d'exemple non restrictif l'acide tartrique, l'acide lactique, l'acide salicylique et des dérivés, l'acide adipique, etc.

Cette liste n'est évidemment pas exhaustive et le chimiste de l'art pourra aisément comprendre que l'invention est destinée à protéger tous les acides organiques et toutes les amines qui sont connus pour inter réagir et conduire ainsi à des amides. Toutes les lipases peuvent être utilisées dans le cadre de l'invention, ainsi que toute autre acyltransférase comme cité précédemment. A titre indicatif sans restrictions on peut citer:

- la lipase de Mucor miehei
- la lipase de pancréas de porc
- la lipase de Rhizopus arrhizus

- la lipase de Candida cylindracea
- la lipase de Geotrichum candidum
- l'acylase d'Aspergillus niger
- la peptidase de Bacillus licheniformis
- la protéinase de Bacillus subtilis
- l'amidase de Brevibacterium
- l'amidase (Novozyme 217)

Les conditions de la réaction en ce qui concerne la durée, le pH de la phase aqueuse lorsqu'elle existe, la température, l'agitation, l'activité de l'eau du catalyseur biologique, la pression ne sont pas critiques mis à part le fait que ces conditions ne doivent pas rendre l'enzyme inactive.

On conçoit donc que les conditions dépendront dans une certaine mesure du type d'enzyme utilisée et de l'utilisation ou non d'un solvant ainsi que du type de solvant.

Le rapport amine/acide organique pourra varier de 90/10 à 10/90. L'invention vient d'être décrite dans son mode de réalisation le plus général afin que l'homme de métier soit à même d'évaleur la portée de celle-ci.

Il est maintenant nécessaire de décrire les variantes préférées de l'invention étant bien entendu que celles-ci ne limitent en aucune façon l'invention à ces variantes.

En premier lieu, il a été dit précédemment qu'un des intérêts de l'invention se situait dans la possibilité d'obtenir une régiosélectivité très forte sur la fonction amine dans le cas d'amines polyfonctionnelles.

Ainsi, le procédé de l'invention trouve une application particulièrement intéressante dans le cas où les amines sont précisément polyfonctionnelles.

Il est possible de doser le catalyseur biologique (enzyme) à la fois en terme de vitesse de réaction et de régiosélectivité par adjonction de sels tels que le borate de sodium, le chlorure de strontium...

A titre d'exemple en ce qui concerne les trois aminopropanols il a été constaté que lorsqu'on oppose l'acide oléique (C18:1) à un aminopropanol le fait d'augmenter le rapport 18:1 / aminopropanol est favorable à la disubstitution dans les trois cas.

Dans le cas de l'amino-1-propanol-2, le rapport mole à mole favorise la N-substitution avec un bon rendement ; il est difficile dans ce cas de synthétiser le disubstitué uniquement. Il semble que, lorsque les fonctions alcool et amine sont vicinales, l'enzyme favorise toujours la substitution de la fonction amine indépendamment de sa position; cependant, sur un plan cinétique, l'amine est substitué plus rapidement en position externe qu'en position interne.

Donc le composé idéal pour la synthèse d'un produit monosubstitué ayant un OH libre est l'amino-1-propanol-2; dans ce cas on observe une acylation régiosélective prononcée car seule la fonction amine en position 1 est acylée. Les deux autres aminopropanols sont convenables pour la synthèse des dérivés disubstitués. En règle générale lorsque les deux fonctions OH et $NH_2$ sont en alpha l'une par rapport à l'autre, la fonction amine est acylée préférentiellement.

En ce qui concerne les amino propanediols, il a été constaté que tous les produits possibles sont synthétisés; cependant dans le cas où les deux fonctions alcools sont vicinales, l'adjonction d'acide borique par exemple, réputé pour complexer les diols vicinaux, on parvient à orienter nettement la réaction vers l'acylation préférentielle de l'amine. Pour illustrer le phénomène on peut admettre le complexe schématique suivant :

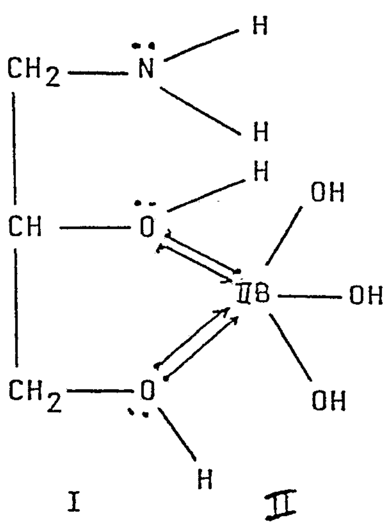

I II

I : Amino-1-propanediol-2-3

II : Acide borique

: Doublet électronique

II : Lacune électronique de l'atome de bore de l'acide borique

La complexation du système vic.diol par l'acide borique est basée sur la tendance des doublets

électroniques des deux atomes d'oxygène à combler la lacune électronique de l'atome de base de l'acide borique.

Le chlorure de strontium joue un rôle analogue sans que l'on puisse expliquer convenablement le mécanisme.

Il est précisé que, lorsqu'on utilise un acide à forte condensation en carbone, l'amine sur laquelle il est prévu de le faire réagir devra de préférence être à faible condensation en carbone et vice versa, sans que ce ne soit une condition absolue.

Il est ainsi possible d'obtenir les précurseurs des quatre types de tensioactifs (cationiques, anioniques, zwitterioniques, et non ioniques).

D'autre part, dans la plupart des cas, l'activité des enzymes est inaltérée après plusieurs cycles ; on peut donc espérer travailler en continu en recyclant les réactifs non combinés.

L'invention est maintenant illustrée par les exemples non limitatifs ci-après.

**Exemples effectués en milieu homogène dans un solvant organique :**

**Exemple 1**

Les conditions de la réaction, effectuée en présence d'un solvant organique : l'hexane, sont données ci-dessous :

acide oléique 200 mg
hexane 15 ml
laurylamine 164 mg
lipase (lipozyme Novo) 100 mg

Température : 60°C
Durée : 24 heures.

Résultat :

On obtient l'amide correspondant avec un taux de conversion de 20 % par rapport à l'acide oléique.

Après 300 heures de réaction, le taux de conversion atteint 70 % par rapport à l'acide oléique.

**Exemple 2**

Identique à l'exemple 1, mais le solvant est le chloroforme (exempt d'alcool) au lieu de l'hexane.

Résultat :

Le taux de conversion, après 24 heures de réaction, est de 10 % par rapport à l'acide oléique.

**Exemple 3**

Identique à l'exemple 1, mais la laurylamine est remplacée par le Dinoram® C (241 mg).

Le Dinoram® C (marque déposée de la société CECA) a la formule :

$R - NH - (CH_2)_3 - NH_2$

dans laquelle

R représente les chaînes grasses du coprah.

Résultat :

Le taux de conversion, après 24 heures de réaction, est de 10 % par rapport à l'acide oléique.

**Exemple 4**

Identique à l'exemple 1, mais la laurylamine est remplacée par l'amine-1-propanol-2 (66 mg) et le solvant est un mélange 30/70 hexane/chloroforme (sans alcool).

Résultat :

Rendement 10 % en dérivé N-acylé.

**Exemple 5**

Identique à l'exemple 1, mais l'acide oléique est remplacé par l'acide ricinoléique.

Résultat :

Le taux de conversion, après 24 heures de réaction, est de 10 % par rapport à l'acide ricinoléique.

**Exemple 6**

Les conditions de la réaction sont les suivantes :
cyclohexane 150 ml
acide palmitique 2 560 mg
monoéthanolamine 611 mg
lipase (lipozyme Novo) 1 000 mg
Température : 37°C
Durée : 16 heures

Après réaction, le précipité est filtré, lavé au cyclohexane afin d'éliminer l'acide palmitique n'ayant pas réagi, dissous dans le chloroforme pour éliminer le lipozyme.

Le produit obtenu est alors recristallisé dans un mélange 1/1 d'éthanol et d'éther éthylique.

Résultat :

Rendement 12 % en N-palmitate de monoéthanolamine.

**Exemple 7**

Les conditions de la réaction sont les suivantes :
cyclohexane 300 ml
acide palmitique 1 026
monoéthanolamine 1 220 mg
lipase (lipozyme Novo) 4 000 mg
Température : 37°C
Durée : 16 heures

La purification est effectuée dans les mêmes conditions que dans l'exemple 6.

Résultat :

Rendement 16 % en N,O-dipalmitate de monoéthanolamine.

**Exemple 8**

Identique à l'exemple 7, mais la monoéthanolamine est remplacée par l'orthophénylènediamine (1 220 mg).

Résultat :

Après 3 jours de réaction, le rendement est de 60 % en pentadécyl-2-benzimidazole.

**Exemple 9**

Identique à l'exemple 7, mais la monoéthanolamine est remplacée par l'amino-2-éthyléthanolamine.

Résultat :

Après 16 heures de réaction, le rendement est de 15 % en N,O-dipalmitate d'aminoéthyléthanolamine, et 11 % en N-palmitate d'aminoéthyléthanolamine.

**Exemple 10**

Identique à l'exemple 7, mais la monoéthanolamine est remplacée par la diéthylènetriamine.

Résultat

Après 5 jours de réaction, le rendement après purification est de 30 % en 1,5-diamide et d'un autre amide de la diéthylènetriamine.

**Exemples effectués en milieu biphasique : phase organique - phase aqueuse**

**Exemple 11**

Les conditions de la réaction sont les suivantes :
- phase organique :
  hexane 3 ml
  acide oléique 112 mg
- phase aqueuse :
  chlorhydrate d'hydroxylamine 138 mg neutralisé à pH 7 à l'aide d'une solution 4 N de soude caustique
- lipase :
  lipozyme Novo 100 mg

Température : 45°C
Durée : 24 heures.

Résultat

Rendement 20 % en acide N-oléylhydroxamique.

**Exemple 12**

Les conditions de la réaction sont les suivantes :
- phase organique :
  acide oléique 112 mg
- phase aqueuse :
  chlorhydrate d'hydroxylamine 138 mg neutralisé à pH 7 à l'aide d'une solution 4 N de soude caustique
  Tampon phosphate 50 millimolaire à pH 7 3 ml
- lipase :
  lipozyme Novo 100 mg

Température : 45°C
Durée : 24 heures.

Résultat

Rendement 75 % en acide N-oléylhydroxamique.

**Exemple 13**

Les conditions de la réaction sont les suivantes :
- phase organique :
  chloroforme (sans alcool) 15 ml
  acide oléique 1 120 mg
- phase aqueuse :
  eau 15 ml
  hydrate d'hydrazine 1 000 mg
- lipase :
  lipozyme Novo 1 000 mg

Température : 45°C
Durée : 24 heures.

Résultat

Rendement 70 % en oléylhydrazine.

**NOTA :**

le chloroforme peut être remplacé par d'autres solvants : dichloroéthane, éther éthylique, méthyltertiobutyléther, etc...

**Exemple 14**

Conditions identiques à celles de l'exemple 12, mais le chlorhydrate d'hydroxylamine est remplacé par l'hydrate d'hydrazine.

Résultat

Rendement 70 % en oléylhydrazine.

**Exemple 15**

Les conditions de la réaction sont les suivantes :
- phase organique :
  hexane 10 ml
  - acide oléique 1 120 mg
- phase aqueuse :
  chlorhydrate d'hydroxylamine 1 380 mg neutralisé à pH 7 à l'aide d'une solution 4 N de soude caustique
- amidase :
  cellules entières de Brevibacterium SP 19 1 000 mg (soit 5 000 mg en poids humide).

Température : 30°C
Durée : 24 heures.

Résultat

Rendement 50 % en acide N-oléylhydroxamique.

**Exemple 16**

Les conditions de la réaction sont les suivantes :
- phase organique :
  acide oléique 345 mg
- phase aqueuse :
  chlorhydrate d'hydroxylamine 2 484 mg neutralisé à pH 7 à l'aide d'une solution 4 N de soude caustique
  Tampon phosphate 50 millimolaires à pH 7 3 ml
- lipase :
  lipozyme Novo 30 mg

Température : 37°C
Durée : 3 jours.

Résultat

Rendement 86 % en acide N-oléylhydroxamique.

**Exemple 17**

Identique à l'exemple 16, mais les 345 mg d'acide oléique sont remplacés par 345 mg d'huile de soja.

Résultat

Rendement 76 % en acide N-hydroxamique des acides gras de l'huile de soja, après 24 heures de réaction.

**Exemple 18**

Identique à l'exemple 16, mais les 345 mg d'acide oléique sont remplacés par 345 mg d'esters méthyliques des acides gras de l'huile de soja.

Résultat

Rendement 60 % en acide N-hydroxamique des acides gras de l'huile de soja, après 24 heures de réaction.

Dans tous les exemples cités, les réactions sont effectuées dans des fioles étanches disposées sur un agitateur vibrant dans une enceinte thermorégulée.

On peut remarquer que les lipases fonctionnent mieux en milieu tamponné et sans solvant organique autre que l'acide gras, alors que les amidases donnent de meilleurs résultats en présence de solvant organique (hexane par exemple) et en présence d'une phase aqueuse très limitée.

**Revendications**

1. Procédé pour la synthèse d'amides gras N-substitués par réaction d'un composé organique présentant une fonction acide terminale et un composé organique présentant une fonction amine terminale caractérisé en ce que ladite réaction est effectuée en présence d'une enzyme de la classe des acyltransférases.

2. Procédé selon la revendication 1, caractérisé en ce que la réaction est effectuée entre un acide de formule RCOOH et une amine de formule $HX-(CH_2)_n-NH_2$ dans laquelle X est O, S ou NH et n est égal à 2 ou 3 et que l'on obtient un produit hétérocyclique de formule

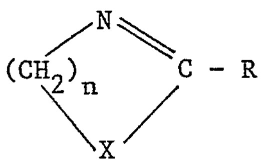

3. Procédé selon la revendication 2,caractérisé en ce que R est une longue chaîne aliphatique.

4. Procédé selon l'une des revendications 1 à 3, caractérisé en ce que la réaction est réalisée en phase homogène dans un solvant organique.

5. Procédé selon l'une des revendications 1 à 3, caractérisé en ce que la réaction est réalisée en phase hétérogène comportant une phase organique et une phase aqueuse.

6. Procédé selon l'une des revendications 1 à 3, caractérisé en ce que la réaction est réalisée en milieu homogène fondu.

7. Procédé selon l'une des revendications 1 à 3, caractérisé en ce que la réaction hétérogène est réalisée en présence d'eau.

Office européen des brevets

# RAPPORT DE RECHERCHE EUROPEENNE

Numero de la demande

EP 88 40 1460

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
| --- | --- | --- | --- |
| A | CHEMICAL ABSTRACTS, vol. 105, no. 19, 10 novembre 1986, page 323, résumé no. 167595w, Columbus, Ohio, US; A. THIERY et al.: "Acyltransferase activity of the wide spectrum amidase of Brevibacterium sp. R312", & J. GEN. MICROBIOL. 1986, 132(8), 2205-8 | 1 | C 12 P 13/02<br>C 12 P 17/00<br>C 12 P 17/10<br>C 12 P 17/14 |
| A | CHEMICAL ABSTRACTS, vol. 77, no. 13, 25 septembre 1972, page 191, résumé no. 85363h, Columbus, Ohio, US; P.H. CLARKE: "Biochemical and immunological comparison of aliphatic amidases produced by Pseudomonas species", & J. GEN. MICROBIOL. 1972, 71(Pt. 2), 241-57 | 1 | |
| A | US-A-3 020 276 (W.B. HUGHES)<br>* Colonnes 6-8 * | 1 | |
| A | EP-A-0 012 371 (BASF)<br>* Revendications; pages 3-8 * | 1 | |

| DOMAINES TECHNIQUES RECHERCHES (Int. Cl.4) |
| --- |
| C 12 P<br>C 12 N<br>C 07 D |

Le présent rapport a été établi pour toutes les revendications

| Lieu de la recherche | Date d'achèvement de la recherche | Examinateur |
| --- | --- | --- |
| LA HAYE | 28-09-1988 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)